# EUROPEAN PATENT SPECIFICATION

(11) **EP 2 450 342 B1**
(45) Date of publication and mention of the grant of the patent: **03.05.2017**
(21) Application number: 12000677.0
(22) Date of filing: 10.01.2005
(51) Int. Cl.: C07C 51/48, C07C 51/47, C07C 63/26

(54) **PROCESS FOR PRODUCTION OF A DRIED CARBOXYLIC ACID CAKE SUITABLE FOR USE IN POLYESTER PRODUCTION**
VERFAHREN ZUR PRODUKTION EINES GETROCKNETEN CARBOXYLSÄUREKUCHENS, DER ZUR VERWENDUNG BEI DER POLYESTERHERSTELLUNG GEEIGNET IST
PROCÉDÉ POUR LA FABRICATION D'UN GÂTEAU D'ACIDE CARBOXYLIQUE SÉCHÉ APPROPRIÉ POUR UNE UTILISATION DANS LA PRODUCTION DE POLYESTER

(30) Priority: 15.01.2004 US 758676
(43) Date of publication of application: 09.05.2012
(62) Divisional of application: 05705384.5
(73) Proprietor: GRUPO PETROTEMEX, S.A. DE C.V., San Pedro Garza Garcia, Nuevo Leon 66265 (MX)
(72) Inventor: Parker, Kenny, Randolph, Afton TN 37616 (US); Lin, Robert, Kingsport TN 37664 (US); Gibson, Philip, Edward, Kingsport TN 37660 (US)
(74) Representative: Tostmann, Holger Carl

(56) References cited:
- EP-A- 0 502 628
- WO-A-92/18453
- DE-A1- 3 128 474
- GB-A- 1 152 577
- GB-A- 1 260 755
- GB-A- 1 388 289
- US-A- 4 201 871
- US-A- 4 334 086
- US-A- 4 357 475
- US-A- 4 939 297
- US-A- 5 200 557

## Description

### FIELD OF INVENTION

The present invention relates to a process by which a dried carboxylic acid cake is obtained from a slurry or cake carboxylic acid product through the use of at least one counter current wash. More specifically, the present invention relates to a process by which a dried terephthalic acid cake suitable as a starting material for polyester or co-polyester production is obtained from a slurry or cake terephthalic acid product through the use of at least one counter current wash.

### BACKGROUND OF THE INVENTION

Pursuant to the goal of making polyethylene terephthalate (PET) and other polyesters or co-polyesters, a great deal of patent literature is dedicated to describing the processes for preparing a dried carboxylic acid cake suitable as starting material. In general, these Inventions describe specific mixing schemes with a purified terephthalic acid solid and liquid ethylene glycol. Additionally, there is substantial body of literature devoted to producing a purified terephthalic acid in the powder form that is suitable for use in producing PET and other polyesters or co-polyesters.

The objective of this invention is to describe a process by which the dried carboxylic acid cake suitable as a starting material for polyester or co-polyester production is obtained from a slurry or cake carboxylic acid product through the use of a counter current solvent wash zone. More specifically, the objective of this invention is to describe a process by which a dried terephthalic acid cake suitable as a starting material for polyester or co-polyester production is obtained from a slurry or cake terephthalic acid product through the use of a counter current solvent wash zone to reduce the amount of fresh solvent used in the process.

Usually, purified terephthalic acid solid is produced in a multi-step process wherein a crude terephthalic acid is produced. Liquid phase oxidation of p-xylene produces crude terephthalic acid. The crude terephthalic acid does not have sufficient quality for direct use as starting material in commercial PET. Instead, the crude terephthalic acid is usually refined to purified terephthalic acid solid.

Usually in terephthalic acid purification processes, the crude terephthalic acid is dissolved in water and hydrogenated for the purpose of converting 4-carboxybenzaldehyde to p-toluic acid, which is a more water soluble derivative, and for the purpose of converting characteristically yellow compounds to colorless derivatives. Significant 4-carboxybenzaldehyde or p-toluic acid in the final purified terephthalic acid product is particularly detrimental to polymerization processes as each can act as a chain terminator during the condensation reaction between terephthalic acid and ethylene glycol in the production of PET. Typical purified terephthalic add contains on a weight basis less than 25 parts per million (ppm)4-carboxybenzaldehyde and less than 150 ppm p-toluic acid,

A number of other processes have been developed where a terephthalic add suitable as starting material for commercial PET production without the use of hydrogenation. Typically, terephthalic production processes involve catalyzed oxidation of p-xylene in an acetic acid solvent followed by filtration and drying of the terephthalic acid.

Typically, terephthalic acid (TPA) produced via catalyzed oxidation of p-xylene in an acetic acid solvent produces a slurry or cake terephthalic acid product that contains residual catalyst (e.g cobalt, manganese, and bromine compounds). In a common method of producing a substantially dry TPA solid from a slurry or cake terephthalic acid product, the slurry or cake terephthalic acid product Is filtered to separate a substantial amount of the acetic acid liquid from the TPA solids. Residual catalyst Is usually separated from the slurry or cake terephthalic acid product by washing (rinsing) the wet cake with catalyst-free acetic acid, water or other solvent. The TPA solid is Isolated by drying.

US 5 200 557 and WO 92/18453 relate to a process for counter-current positive displacement of an aliphatic carboxylic acid of 1 to 5 carbon atoms from a filter cake of an aromatic polycarboxylic acid containing the aliphatic carboxylic acid, wherein mother liquor retained by the aromatic polycarboxylic acid has a concentration of the aliphatic carboxylic acid of 5000 ppmw, or less, based upon weight of the aromatic polycarboxylic acid present. This method is useful for the manufacture of crude terephthalic acid which is used after purification for the preparation of polyesters used for the manufacture of fabrics, fibers and plastic bottles.

US 4 201 871 relates to a process for recovering highly pure terephthalic acid crystals from a suspension containing terephthalic acid product obtained by the liquid phase oxidation of para-substituted aromatic compounds in the presence of oxidizing catalyst. The process comprises bringing said suspension into continuous countercurrent contact with ascending hot acetic acid containing water in a single tower under the conditions of specified temperature, specified ascending rate of the acetic acid containing water and specified residence time of the suspension, to replace the mother liquor of the suspension with said acetic acid, and recovering the mother liquor as an overflow, while recovering terephthalic acid as an underflow in the form of suspension of highly purified crystals and, if necessary, allowing the suspension to stay in a stirring tank.

EP 0 502 628 relates to a terephthalic acid slurry in acetic acid which is produced by oxidizing p-xylene in acetic acid, removing water by evaporation of a stream of water and acetic acid, and returning acetic acid to the oxidation step. The terephthalic acid is separated from the reaction medium in a first zone to leave a deposit on a band, the deposit is washed with a first aqueous medium in a second zone, removed from the band in a third zone, and admixed with a second aqueous medium. Reaction medium is passed from the first zone to the oxidation step and terephthalic acid is recovered, preferably after further purification.

GB 1 152 577 relates to purification of aromatic polycarboxylic acids. An aromatic polycarboxylic acid containing undesirable aldehyde and other impurities is purified by a process which comprises contacting a solution of the impure acid in demineralized water in the liquid phase at an elevated temperature and pressure and in the presence of H₂ with a Pd-containing catalyst under mild hydrogenation conditions, and recovering purified acid. The aromatic polycarboxylic acid may be obtained by liquid phase catalytic oxidation of polyalkyl aromatic hydrocarbons. Specified polycarboxylic, acids which may be purified are terephthalic, isophthalic, trimesic, trimellitic, mellitic and naphthalene dicarboxylic acids. The hydrogenation is suitably carried out at a temperature in the range of about 450-600 F in an inert polar solvent either as a batch process under static conditions or as a continuous process maintaining a concurrent or countercurrent flow of H₂ through the catalyst chamber. A sufficient pressure may be maintained in a reactor by using a mixture of H₂ and an inert gas, e.g. N₂. The product may be obtained from the reaction mixture by filtering and crystallizing from the filtrate.

US 4 334 086 relates to a process for the continuous production of terephthalic acid from p-xylene, comprising the steps of oxidizing p-xylene in a first oxidation zone in the presence of not more than about 10 weight % of water and a cobalt/manganese catalyst up to a conversion of about 15% terephthalic acid; oxidizing the partially-oxidized compounds in a second oxidation zone in the presence of an additional amount of water up to about 20 to 70% by weight and in the presence of an additional amount of the catalyst up to a conversion of about 50% by weight terephthalic acid; stripping p-xylene vapors from the second oxidation zone; separating crystals of crude terephthalic acid from the effluent from the second oxidation zone from the soluble components of the effluent; countercurrently washing the separated crystals with fresh water to produce a slurry; recycling a part of soluble components to the second oxidation zone, and another part of the first oxidation zone to provide the latter with heavy metal catalyst; and recovering purified terephthalic acid by recyrstallizing the crystals contained in the slurry.

GB 1 388 289 relates to the production and recovery of terephthalic acid, wherein the process for the preparation of terephthalic acid from an aqueous solution of dipotassium terephthalate comprises (i) reacting the dipotassium terephthalate in said solution with benzoic acid to produce an aqueous slurry containing dissolved potassium benzoate and undissolved benzoic acid, potassium hydrogen terephthalate, and terephthalic acid; (ii) countercurrently contacting said slurry in an elongated zone with a stream of water, the temperature in said zone adjacent the upstream end being maintained at a temperature in the range 38-66 °C and adjacent the downstream end at a lower temperature, such lower temperature being in the range 10-38 °C; (iii) recovering from said upstream end an aqueous solution of potassium benzoate and from said downstream end an aqueous slurry of benzoic acid, potassium hydrogen terephthalate and terephthalic acid; (iv) countercurrently contacting the slurry recovered in step (iii) in a second elongated contacting zone with a second stream of water, the temperature in said second zone adjacent the upstream end being maintained at a temperature in the range 66-121 °C, and adjacent the downstream end at a lower temperature, such lower temperature being in the range 24-66 °C; (v) recovering from the upstream end of said second zone a second aqueous solution of potassium benzoate and from the downstream and an aqueous slurry of terephthalic acid; and (vi) separating the terephthalic acid from the slurry recovered in step (v).

GB 1 260 755 relates to the production of aromatic carboxylic acids, wherein a process for the production of an aromatic carboxylic acid by the hydrolysis of an aromatic nitrile comprises (a) maintaining an aqueous slurry of the nitrile in contact with a catalyst which is an alkali metal or alkaline earth metal hydroxide, carbonate or salt of an aromatic carboxylic acid or ammonium salt of an aromatic carboxylic acid at a temperature in the range 300-600 °F for a period sufficient to hydrolyze a substantial portion of the nitrile, (b) stripping ammonia from the hydrolysate by countercurrently contacting the hydrolysate with steam and (c) cooling the stripped hydrolysate to a temperature at which the aromatic carboxylic acid separates out. In the examples terephthalic acid is prepared from terephthalonitrile.

US 4 357 475 relates to a process wherein a reaction mixture obtained by oxidizing p-xylene in the presence of water as diluent, at a temperature comprised between 140 °C and 220 °C, and consisting of terephthalic acid crystals in suspension in an aqueous solution comprising unreacted p-xylene, intermediate oxidation products thereof, the heavy-metal catalyst and water is introduced in the upper part of a sedimentation column wherein the acid is separated by gravity and is washed with a counter current of water introduced near the bottom of said column, the temperature of the washing zone being higher than the minimum value Tw given by the equation Tw=144+0.225 TR, wherein TR is the oxidation temperature.

US 4 939 297 relates to a method for removing impurities from an oxidative terephthalic acid synthesis mother liquor containing acetic acid, water, corrosion metals, a metal catalyst and organic impurities, comprising the steps of (a) removing from the mother liquor by evaporation from 50-95% of the acetic acid and water contained therein; (b) adding an amount of water to the concentrated mother liquor sufficient to dissolve the metal catalyst and form an aqueous mixture; (c) extracting the aqueous mixture by counter-current extraction with a substantially water-insoluble organic solvent, to produce a lighter phase containing the organic solvent, a minor amount of the water, acetic acid and the organic impurities and a heavier phase containing a major amount of the water, corrosion metals and the metal catalyst, and; (d) removing the corrosion metals from the heavier phase by heating and filtering, and; (e) removing the organic impurities from the lighter phase by distillation,

DE 31 28 474 relates to a process for countercurrent washing of finely divided crude terephthalic acid using a washing liquid of substantially different density in a washing tower.

In the present invention, a novel process has been discovered resulting in less solvent used than currently employed processes. In the conventional approach toward producing terephthalic acid via catalyzed oxidation of p-xylene in an acetic acid solvent, a slurry or cake terephthalic
acid product is filtered, washed, then dried to produce a terephthalic acid powder suitable as starting material for PET production.

In accordance with the present invention, the slurry or cake terephthalic acid product is filtered to produce a terephthalic acid cake with solvent and a TPA solvent mother liquor stream. The terephthalic acid cake with solvent is then washed (rinsed) with water to recover residual metal catalyst material and to produce a water-wet terephthalic acid cake and an TPA solvent/water by-product liquor. The water-wet terephthalic acid cake Is then dried to produce a dried terephthalic acid cake suitable as starting material in a commercial PET process. In this embodiment of the invention at least one counter current wash is utilized.

Specifically, the present invention relates to a process for producing a dried carboxylic acid cake (170), said process comprising:
(a) removing, in a solid-liquid separation zone (40), impurities from a carboxylic acid slurry (30) to form a slurry or cake product (70) and a mother liquor stream (60), wherein said carboxylic acid slurry (30) is produced by oxidizing, in an oxidation zone, an aromatic feedstock; and wherein said solid-liquid separation zone (40) comprises at least one solid-liquid separator which is operated at temperatures between 50 °C and 200 °C;
(b) adding solvent to said slurry or cake product (70) in a counter current solvent wash zone (80) to produce a carboxylic acid cake with solvent (110) and a solvent mother liquor stream (100); wherein the solvent comprises an aliphatic monocarboxylic acid containing from 2 to 6 carbon atoms, or benzoic acid, and mixtures of these compounds with water; and wherein the counter current solvent wash zone (80) comprises at least one solid-liquid separation device which is operated within a temperature range of from 40 °C to 155 °C;
(c) optionally, adding water in a counter current water wash zone (120) to said carboxylic cake with solvent (110) to produce a water-wet carboxylic acid cake (150) and a solvent/water by-product liquor stream (140); wherein said counter current wash zone comprises a solid-liquid separation device which is operated within a temperature range from 40 to 155 °C;
(d) drying said water-wet carboxylic acid cake (150) or said carboxylic acid cake with solvent (110) in a drying zone (160) to form said dried carboxylic acid cake (170).

By utilizing a counter current solvent wash zone the amount of solvent used can be reduced substantially as compared to a process without counter current washing In addition, by utilizing at least one counter current wash may result in reduction of equipment size and energy as compare to a TPA production process without a counter current wash.

### SUMMARY OF THE INVENTION

The present invention relates to a process by which a dried carboxylic acid cake is obtained from a slurry or cake carboxylic acid product. More specifically, the present invention relates to a process for the production of a dried terephthalic acid cake suitable as feedstock for the
production of commercial PET. The resulting process utilizes less solvent than currently employed processes that do not utilize a counter current solvent wash zone.

It is an object of this invention to provide a process for producing a dried carboxylic acid cake from a slurry or cake carboxylic acid product though the use of at least one counter current wash.

It is another object of this invention to provide a process for producing a dried terephthalic acid cake from a slurry or cake terephthalic acid product

It is another object of this invention to provide a process for producing a dried terephthalic acid cake from a terephthalic acid solvent slurry or cake through the use of a counter current solvent wash zone.

The present invention provides a process for producing a dried carboxylic acid cake, the process comprising:
(a) removing, in a solid-liquid separation zone (40), impurities from a carboxylic acid slurry (30) to form a slurry or cake product (70) and a mother liquor stream (60), wherein said carboxylic acid slurry (30) is produced by oxidizing, in an oxidation zone, an aromatic feedstock; and wherein said solid-liquid separation zone (40) comprises at least one solid-liquid separator which is operated at temperatures between 50 °C and 200 °C;
(b) adding solvent to said slurry or cake product (70) in a counter current solvent wash zone (80) to produce a carboxylic acid cake with solvent (110) and a solvent mother liquor stream (100); wherein the solvent comprises an aliphatic monocarboxylic acid containing from 2 to 6 carbon atoms, or benzoic acid, and mixtures of these compounds with water; and wherein the counter current solvent wash zone (80) comprises at least one solid-liquid separation device which is operated within a temperature range of from 40 °C to 155 °C;
(c) optionally, adding water in a counter current water wash zone (120) to said carboxylic cake with solvent (110) to produce a water-wet carboxylic acid cake (150) and a solvent/water by-product liquor stream (140); wherein said counter current wash zone comprises a solid-liquid separation device which is operated within a temperature range from 40 to 155 °C;
(d) drying said water-wet carboxylic acid cake (150) or said carboxylic acid cake with solvent (110) in a drying zone (160) to form said dried carboxylic acid cake (170).

In one embodiment of this invention, a process for producing a dried terephthalic acid cake Is provided, the process comprises:
(a) removing In a solid-liquid separation zone Impurities from a terephthalic acid scurry to form a slurry or cake terephthalic acid product and a mother liquor stream;
(b) adding solvent in a counter current solvent wash zone to the slurry or cake terephthalic acid product to produce a terephthalic acid cake with solvent and a solvent mother liquor stream;
(c) optionally, adding water in a counter current water wash zone to the terephthalic acid cake with solvent to produce a water-wet terephthalic acid cake and a solvent/water by product liquor stream;
(d) drying the water-wet carboxylic acid cake in a drying zone to form the dried carboxylic acid cake.

These objects, and other objects, will become more apparent to others with ordinary skill in the art after reading this disclosure.

### BRIEF DESCRIPTION OF THE DRAWINGS

Figure 1 illustrates one embodiment of this Invention, a process for producing a dried carboxylic acid cake,
Figure 2 Illustrates another embodiments of this invention, a process for producing a dried carboxylic acid cake by utilizing a liquor exchange zone.
Figure 3 Illustrates another embodiment of this invention, a process for, producing a dried carboxylic acid cake by utilizing a counter current solvent-water liquor exchange zone.
Figure 4 illustrates another embodiment of this invention, a process for producing a dried carboxylic acid cake by utilizing a solvent liquor exchange zone.

### DESCRIPTION OF THE INVENTION:

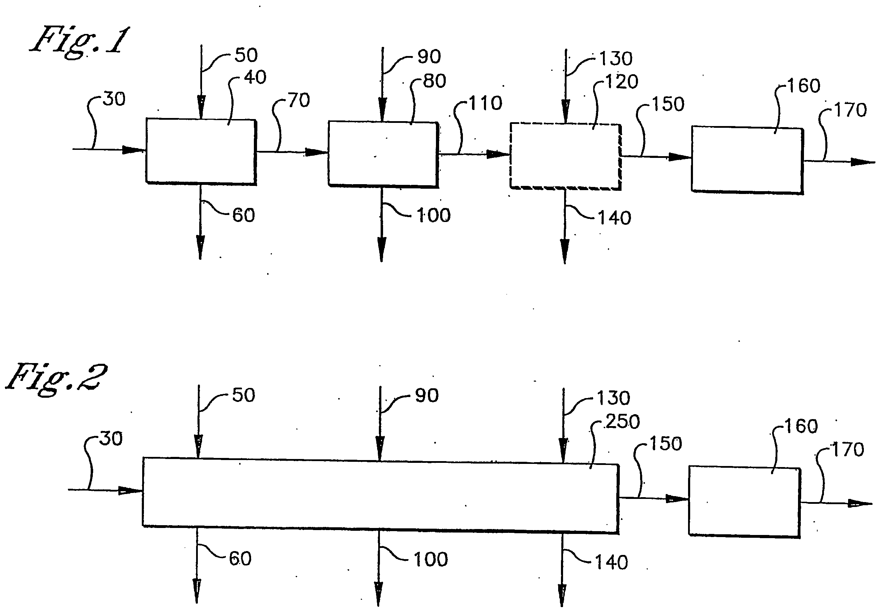

In an embodiment of this invention shown in Figure 1, a process for producing a dried carboxylic acid cake 170 is provided. The process comprises:
Step (a) comprises removing impurities from a carboxylic acid slurry 30 in an solid-liquid displacement zone 40 to form a slurry or cake carboxylic acid product 70 and a mother liquor stream 60;

A carboxylic acid slurry comprises at least one carboxylic acid, catalyst, at least one solvent, and impurities is introduced via lines not shown. The impurities typically comprise at least one or more of the following compounds: 4-carbaxybenzaldehyde(4-CBA), trimellitic acid(TMA), and 2,6-dicarboxyfluorenone(2,6-DCF). Suitable solvents include, but are not limited to, aliphatic mono-carboxylic acids, preferably containing 2 to 6 carbon atoms, or benzoic add and mixtures thereof and mixtures of these compounds with water. Preferably the solvent is acetic acid mixed with water, in a ratio of 5:1 to 99:1, preferably between 8:1 and 49:1, Throughout the specification acetic acid will be referred to as the solvent. However, It should be appreciated that other suitable solvents, such as those disclosed previously, may also be utilized. The solvent typically comprises acetic acid, but can be any solvent that has been previously mentioned.

The carboxylic acid slurry can be produced by oxidizing in a oxidation zone an aromatic feed stock. In one embodiment the aromatic feedstock comprises paraxylene. The oxidation zone comprises at least one oxidation reactor, and the carboxylic acid slurry comprises at least one carboxylic acid. The oxidation reactor can be operated at temperatures between 120°C and 250°C. Preferably 140°C to 170°C. Typically the aromatic feed stock comprises paraxylene and the carboxylic acid comprises terephthalic acid. In one embodiment of the invention the oxidation zone comprises a bubble column.

Therefore, for example, when terephthalic acid is utilized, the carboxylic acid slurry 30 would be referred to as terephthalic acid slurry and the dried carboxylic acid cake 170 would be referred to as a dried terephthalic acid cake.

Carboxylic acids include any carboxylic acid produced via controlled oxidation of an organic precursor compound. For example, carboxylic acids include aromatic dicarboxylic acids preferably having 8 to 14 carbon atoms, aliphatic dicarboxylic acids preferably having 4 to 12 carbon atoms, or cycloaliphatic dicarboxylic acids preferably having 8 to 12 carbon atoms. Other examples of suitable carboxylic acids include, but are not limited to, terephthalic acid, benzoic, p-toulic, isophthalic acid, trimellitic add, naphthalene dicarboxylic acid, cyclohexanedicarboxylic acid, cyclohexanediacetic acid, diphenyl-4,4'-dicarboxylic acid, diphenyl-3,4'-dicarboxylic acid. 2,2,-dimethyl-1,3-propandiol dicarboxylic acid, succinic acid, glutaric acid, adipic acid, azelaic acid, sebacic acid, and mixtures thereof.

Terephthalic acid slurry is conventionally synthesized via the liquid phase oxidation of paraxylene in the presence of suitable oxidation catalyst. Suitable catalysts include, but are not limited to, cobalt, manganese and bromine compounds, which are soluble in the selected solvent. In one embodiment of the invention the catalyst comprises cobalt, bromine and manganese. The cobalt and manganese combined can be in concentrations of 100 ppm to 2700 ppm by weight in the liquor. The bromine can be In concentrations of 1000 ppm to 2500 ppm by weight in the liquor.

The carboxylic acid slurry is fed to a solid-liquid displacement zone 40 capable of removing a portion of the liquid contained in the carboxylic acid slurry 30 to produce a slurry or cake carboxylic acid product in conduit 70. The removal of a portion of the liquid to produce a slurry or cake carboxylic acid product in conduit 70 can be accomplished by any means known In the art. A portion means at least **5**% by weight of the liquid Is removed. Typically, the solid-liquid displacement zone 40 comprises a solid-liquid separator that is selected from the group consisting of a decanter centrifuge, rotary disk centrifuge, belt filter, rotary vacuum filter.

In accordance with the present invention, the carboxylic acid slurry in conduit 30 is fed to the solid-liquid displacement zone 40 comprising at least one solid-liquid separator. In accordance with the present invention, the solid-liquid separator(s) is/are operated at temperatures between 50°C to 200°C, preferably 140°C to 170°C. The solid-liquid separator(s) can be operated at pressures between 0 Pa (0 psig) to 1400000 Pa (200 psig). The solid-liquid separator in the solid-liquid displacement zone 40 may be operated in continuous or batch mode, although it will be appreciated that for commercial processes, the continuous mode is preferred,

The Impurities are displaced from the solid-liquid displacement zone **40** into a mother liquor stream and withdrawn via line **60.** In one embodiment of the Invention, additional solvent is fed to the solid-liquid displacement zone **40** via line **50** to reslurry the carboxylic acid slurry **30** and form a slurry or cake carboxylic acid product **70.** When a terephthalic acid slurry is utilized in the solid-liquid separation zone **40,** a slurry or cake terephthalic acid product is produced, The slurry or cake terephthalic acid product typically comprises terephthalic acid and acetic acid. The mother liquor **60** is withdrawn from solid-liquid displacement zone **40** via line **60** and comprises a solvent, typically acetic acid, catalyst, and bromine compounds. The mother liquor in line **60** may either be sent to a process for separating impurities from oxidation solvent via lines not shown or recycled to the catalyst system via lines not shown. One technique for impurity removal from the mother liquor **60** commonly used In the chemical processing industry is to draw out or "purge" some portion of the recycle stream. Typically, the purge stream is simply disposed of or, if economically Justified, subjected to various treatments to remove undesired impuritles while recovering valuable components, Examples of impurity removal processes include U.S. Patent 4,939,297 and U.S. Patent 4,3513,319,
Step (b) comprises removing in a counter current solvent wash zone **80** residual Impurities from a slurry or cake carboxylic acid product **70** to form a carboxylic acid cake with solvent **110** and a solvent mother liquor stream **100.**

Conduit **70** contains a slurry or cake carboxylic acid product **70** comprising a carboxylic acid, residual impurities and a solvent. The residual impurities comprise residual catalyst (typically but not limited to cobalt, manganese, or bromine). Suitable solvents include, but are not limited to, aliphatic mono-carboxylic acids, preferably containing 2 to 6 carbon atoms, or benzoic acid and mixtures thereof and mixtures of these compounds with water. Preferably, the solvent is comprised of mainly acetic acid and/or some water. The ratio of acetic acid to water can range from 50;50 to 99:1 acetic acid to water by mass, more preferably in the range of 85:15 to 98.2. and most preferably in the range of 90:10 to 97:3. Suitable carboxylic acids Include by are not limited to terephthalic acid, isophthalic acid, naphthalene dicarboxylic acid, trimellitic acid, and mixtures thereof.

The slurry or cake carboxylic acid product 70 is in the range of 10-90% by weight carboxylic acid. Preferably the slurry or cake carboxylic acid product **70** is in the range of 25-40% by weight carboxylic acid for a slurry and in the range of 70-90% by weight for the cake product. Most preferably, the slurry or cake carboxylic acid product **70** is in the range of 30-40% by weight carboxylic acid. The slurry or cake carboxylic acid product in conduit **70** is then introduced into a counter current solvent wash zone **80,** wherein a substantial portion of solvent is recovered in the solvent mother liquor stream In conduit **100.** The solvent mother liquor **102** comprises a substantial portion of the solvent. Additional solvent can be added via conduit **90** counter current to the flow of the slurry or cake carboxylic acid product **70** in the counter current solvent wash zone **80,** The amount of stages of counter current wash can be any amount of stages necessary to produce the carboxylic cake with solvent to the desired purity, Typically, the amount of stages In the counter current wash can be 1 to 8, preferably 2 to 6, most preferably 2 to 4. For wash with more than one stage, counter current flow is preferable.

The counter current solvent wash zone 80 comprises at least one solid-liquid separation device capable of efficiently separating solids and liquids. The solid-liquid separation device can typically be comprised of, but not limited to, the following types of devices: centrifuges, cyclones, rotary drum filters, belt filters, press filters, etc. In accordance with the present invention, the counter current solvent wash zone 80 comprises at least one solid-liquid separation device(s) 110 which operates within a temperature range of from 40°C to 155°C. Preferably the solid-liquid separation device(s) 110 can operate within a temperature range of from 80°C to 150°C. Most preferably the solid-liquid separation device(s) 110 operates within a temperature range of from 90°C to 150°C. A carboxylic acid cake with solvent **110,** is produced wherein the moisture composition of the carboxylic add cake with solvent **110** can be in the range of 0.5-30% by weight moisture, preferably in the range of 1-20% moisture, most preferably in the range of 1-10% moisture. Optionally, the residual solvent can be removed by a gas displacement step to minimize solvent contamination with wash. When the carboxylic acid is terephthalic acid and the solvent is acetic acid a terephthalic acid cake with acetic acid is produced.
Step (c) comprises optionally removing a substantial portion of a solvent in a counter current water wash zone **120** from the carboxylic acid cake with solvent **110** to form a water-wet carboxylic acid cake **100** and a solvent/water byproduct liquor stream **140.**

The carboxylic acid cake with solvent **110.** is then subjected to a wash or "rinsing" with water or substantially water with residual amounts of solvent in the counter current water wash zone **120,** wherein a substantial portion of the solvent is replaced with water to form a water-wet carboxylic acid cake **150.** The water-wet carboxylic acid cake **150,** is preferably in the range of 0.5% to 30% moisture, more preferably in the range of 1 to 20% moisture, and most preferably in the range of 1 % to 10% moisture. The residual moisture of the water-wet carboxylic acid cake **150,** should contain less than 2% solvent on a mass basis. Additionally, the water-wet carboxylic acid cake **150** should contain less than 1% of any metals, preferably less than 100 ppm by weight, most preferably less than 10 ppm by weight , typically used as catalysts in p-xylene oxidation, In the slurry or cake carboxylic acid product In conduit **70,** should remain in the water-wet carboxylic acid cake **150.** Examples of metals include but are not limited to cobalt, and manganese.

Wash water is introduced Into the counter current water wash zone **120** via conduit **130.** The wash water should be, on a continuous basis, comprise a mass feed rate in ratio with the solids in the carboxylic cake with solvent **110** In the range of 0.1:1 to 1,5;1, preferably in the range of 0.1:1 to 0.6:1, most preferably in the range of 0.2:1 to 0.4:1. There are no limitations on the temperature or pressure of the wash water including the use of vaporized water, steam, or a combination of water and steam, as wash. In one embodiment of the invention, wash water is introduced counter current to the carboxylic acid cake with solvent.

Additional wash water can be added via conduit **130** counter current to the flow of the carboxylic acid cake with solvent **110** in the counter current water wash zone **120.** The amount of stages of counter current wash can be any amount of stages necessary to produce the water wet carboxylic acid cake to the desired purity. Typically, the amount of stages in the counter current wash can be 1 to 8, preferably 2 to 6, most preferably 2 to 4.

The counter current water wash zone comprises a solid-liquid separation device **120** can typically be comprised of, but not limited to, the following types of devices: centrifuge, cyclones, rotary drum filters, belt filters, press filters, The solid-liquid separation device can be operated within a temperature range of from 40°C to 155°C. Preferably, the second solid-liquid separation device can operate within a temperature range of from 80°C to 150°C. Most preferably, the second solid-liquid separation device can operate within a temperature range of from 90°C to 150°C

Optionally, the solvent/water byproduct liquor from the counter current water wash zone **120,** is segregated from the solvent mother liquor stream produce by the counter current solvent wash zone **80.**
Step (d) comprises drying the water-wet carboxylic acid cake **150** in a drying zone **160** to produce a dried carboxylic acid product **170.**

The water wet carboxylic acid cake **150** is withdrawn from the counter current water wash zone **120** or the counter current solvent wash zone **80** and fed to the drying zone **160.** A portion of the solvent or water and remaining catalyst and impurities Is separated, and the dried carboxylic acid cake is withdrawn via line **170.**

The drying zone **160** comprises a filter suitable for recovering the solid carboxylic acid and a dryer. The filtration can be accomplished by any means known in the art. For example, a rotary vacuum filter can be used for the filtration to produce a filtration cake, The filtration cake goes through an initial solvent removal step, is then rinsed with acid wash to remove residual catalyst, and can solvent removed again before sent to the dryers. The drying of the filter cake can be accomplished by any means known in the art that's capable of evaporating at least 10% of the volatiles remaining in the filter cake to produce the carboxylic acid product. For example, a Single Shaft Porcupine® Processor dryer can be used.

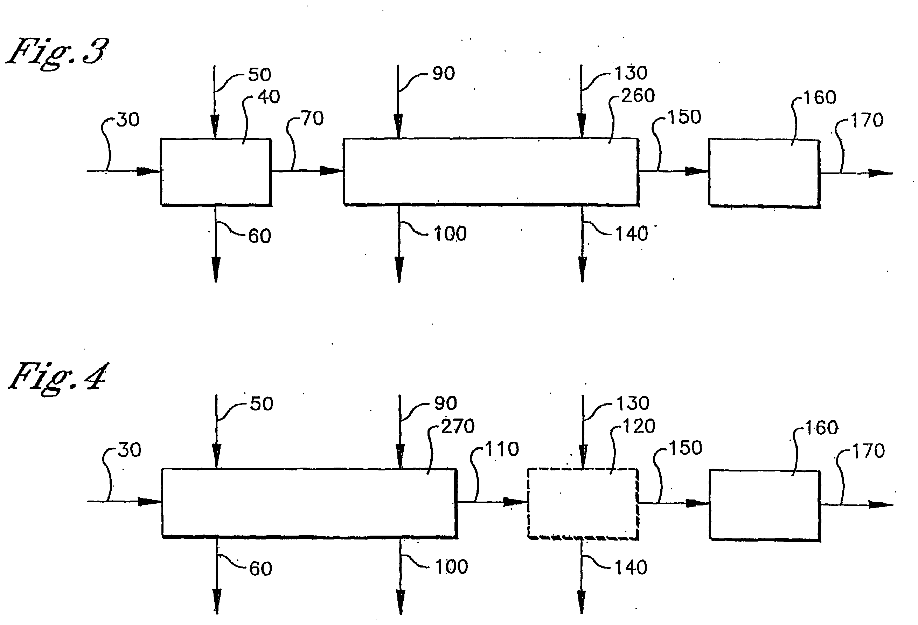

In other embodiments of this invention step (a), step (b) and step (c) can be combined into one zone known as the liquor exchange zone **250** as shown in figure 2, The liquor exchange zone **250** comprises at least one solid-liquid separation device capable of performing the combined function of the solid-liquid separation zone **40,** the counter current solvent wash zone **80** and the counter current water wash zone **120** as previously described. Step (b) and step (c) can also be combined into one zone known as the counter current solvent-water liquor exchange zone **260** as shown in Figure 3. Finally step (a) and step (b) can be combined into one zone known as the solvent liquor exchange zone **270** as show in Figure 4. In each of the above embodiments comprises at least one solid-liquid separation device capable of performing the functions of the combined zones as previously described. Examples of devices that can be used in the liquor exchange zone **250,** or the solvent-water liquor exchange zone **260,** or the solvent liquor exchange zone **270** included but are not limited to, the following type of devices centrifuges, cyclones, filters, or combination thereof.

## Claims

1. A process for producing a dried carboxylic acid cake (170), said process comprising:
(a) removing, in a solid-liquid separation zone (40), impurities from a carboxylic acid slurry (30) to form a slurry or cake product (70) and a mother liquor stream (60), wherein said carboxylic acid slurry (30) is produced by oxidizing, in an oxidation zone, an aromatic feedstock;
and wherein said solid-liquid separation zone (40) comprises at least one solid-liquid separator which is operated at temperatures between 50 °C and 200 °C;
(b) adding solvent to said slurry or cake product (70) in a counter current solvent wash zone (80) to produce a carboxylic acid cake with solvent (110) and a solvent mother liquor stream (100); wherein the solvent comprises an aliphatic monocarboxylic acid containing from 2 to 6 carbon atoms, or benzoic acid, and mixtures of these compounds with water;
and wherein the counter current solvent wash zone (80) comprises at least one solid-liquid separation device which is operated within a temperature range of from 40 °C to 155 °C;
(c) optionally, adding water in a counter current water wash zone (120) to said carboxylic cake with solvent (110) to produce a water-wet carboxylic acid cake (150) and a solvent/water by-product liquor stream (140);
wherein said counter current wash zone comprises a solid-liquid separation device which is operated within a temperature range from 40 to 155 °C;
(d) drying said water-wet carboxylic acid cake (150) or said carboxylic acid cake with solvent (110) in a drying zone (160) to form said dried carboxylic acid cake (170).

2. The process according to claim 1 wherein said carboxylic acid is selected from a group consisting of terephthalic acid, isophthalic acid, naphthalene dicarboxylic acid, trimellitic acid and mixtures thereof.

3. The process according to claim 1 wherein said carboxylic acid is terephthalic acid.

4. The process according to claim 3 wherein said crude carboxylic acid slurry (30) comprising terephthalic acid, catalyst, acetic acid, and impurities is withdrawn at a temperature between 110°C and 200°C from an oxidation zone.

5. The process according to claim 1 for producing a dried terephthalic acid cake, said process comprising:
(a) removing in a solid-liquid separation zone (40) impurities from a terephthalic acid slurry (30) to form a slurry or cake terephthalic acid product (70) and a mother liquor stream (60);
(b) adding solvent in a counter current solvent wash zone (80) to said slurry or cake terephthalic acid product (70) to produce a terephthalic acid cake with solvent (110) and a solvent mother liquor stream (100);
(c) optionally, adding water in a counter current water wash zone (120) to said terephthalic acid cake with solvent (110) to produce a water-wet terephthalic acid cake (150) and a solvent/water by-product liquor stream (140);
(d) drying said water-wet terephthalic acid cake (150) or said terephthalic acid cake with solvent (110) in a drying zone (160) to form said dried terephthalic acid cake (170).

6. The process according to claim 5 wherein said crude carboxylic acid slurry (30) comprising terephthalic acid, catalyst, acetic acid, and impurities is withdrawn at a temperature between 110°C and 200°C from an oxidation zone.

7. The process according to claim 5 wherein said counter current water wash zone (80) comprises from 2 to 4 stages of water counter current washes.

8. The process according to claim 5 wherein said counter current solvent wash zone (80) comprises from 2 to 4 stages of solvent counter current washes.

9. The process according to claim 7 wherein said counter current solvent wash zone (80) comprises from 2 to 4 stages of solvent counter current washes.

## Patentansprüche

1. Verfahren zur Herstellung eines getrockneten Carbonsäurekuchens (170), wobei das Verfahren aufweist:
(a) ein Entfernen von Verunreinigungen aus einer Carbonsäureaufschlämmung (30) in einer Fest-Flüssig-Trennzone (40), um ein Aufschlämmungs- oder Kuchenprodukt (70) und einen Mutterlaugenstrom (60) zu bilden, wobei die Carbonsäureaufschlämmung (30) hergestellt wird, indem ein aromatisches Ausgangsmaterial in einer Oxidationszone oxidiert wird,
und wobei die Fest-Flüssig-Trennzone (40) mindestens einen Fest-Flüssig-Separator aufweist, der bei Temperaturen zwischen 50 °C und 200 °C betrieben wird,
(b) ein Hinzufügen eines Lösungsmittels an das Aufschlämmungs- oder Kuchenprodukt (70) in einer Gegenstrom-Lösungsmittel-Waschzone (80), um einen Carbonsäurekuchen mit dem Lösungsmittel (110) und einen Lösungsmittel-Mutterlaugenstrom (100) herzustellen, wobei das Lösungsmittel eine aliphatische Monocarbonsäure, die 2 bis 6 Kohlenstoffatome aufweist, oder eine Benzoesäure und Mischungen dieser Komponenten mit Wasser aufweist, und wobei die Gegenstrom-Lösungsmittel-Waschzone (80) mindestens eine Fest-Flüssig-Trennvorrichtung aufweist, die in einem Temperaturbereich von 40 °C bis 155 °C betrieben wird,
(c) wahlweise ein Hinzufügen von Wasser in einer Gegenstrom-Wasser-Waschzone (120) an den Carbonsäurekuchen mit dem Lösungsmittel (110), um einen wassernassen Carbonsäurekuchen (150) und einen Lösungsmittel/Wasser-Nebenprodukt-Flüssigkeitsstrom (140) herzustellen,
wobei die Gegenstrom-Wasser-Waschzone eine Fest-Flüssig-Trennvorrichtung aufweist, die in einem Temperaturbereich von 40 °C bis 155 °C betrieben wird,
(d) ein Trocknen des wassernassen Carbonsäurekuchens (150) oder des Carbonsäurekuchens mit dem Lösungsmittel (110) in einer Trocknungszone (160), um den getrockneten Carbonsäurekuchen (170) zu bilden.

2. Verfahren nach Anspruch 1, wobei die Carbonsäure aus einer Gruppe ausgewählt worden ist, die aus einer Terephthalsäure, einer Isophthalsäure, einer Naphthalindicarbonsäure, einer Trimellithsäure und deren Mischungen besteht.

3. Verfahren nach Anspruch 1, wobei die Carbonsäure eine Terephthalsäure ist.

4. Verfahren nach Anspruch 3, wobei die unaufgereinigte Carbonsäureaufschlämmung (30), die eine Terephthalsäure, einen Katalysator, eine Essigsäure und Verunreinigungen enthält, bei einer Temperatur zwischen 110 °C und 200 °C aus einer Oxidationszone entnommen wird.

5. Verfahren nach Anspruch 1 zur Herstellung eines getrockneten Terephthalsäurekuchens, wobei das Verfahren aufweist:
(a) ein Entfernen von Verunreinigungen aus einer Terephthalsäureaufschlämmung (30) in einer Fest-Flüssig-Trennzone (40), um ein Aufschlämmungs- oder Terephthalsäure-Kuchenprodukt (70) und einen Mutterlaugenstrom (60) zu bilden,
(b) ein Hinzufügen eines Lösungsmittels in einer Gegenstrom-Lösungsmittel-Waschzone (80) an das Aufschlämmungs- oder Terephthalsäure-Kuchenprodukt (70), um einen Terephthalsäurekuchen mit dem Lösungsmittel (110) und einem Lösungsmittel-Mutterlaugenstrom (100) zu bilden,
(c) wahlweise ein Hinzufügen von Wasser in einer Gegenstrom-Wasser-Waschzone (120) an den Terephthalsäurekuchen mit dem Lösungsmittel (110), um einen wassernassen Terephthalsäurekuchen (150) und einen Lösungsmittel/Wasser-Nebenprodukt-Flüssigkeitsstrom (140) zu bilden,
(d) ein Trocknen des wassernassen Terephthalsäurekuchens (150) oder des Terephthalsäurekuchens mit dem Lösungsmittel (110) in einer Trocknungszone (160), um den getrockneten Terephthalsäurekuchen (170) zu bilden.

6. Verfahren nach Anspruch 5, wobei die unaufgereinigte Carbonsäureaufschlämmung (30), die eine Terephthalsäure, einen Katalysator, eine Essigsäure und Verunreinigungen enthält, bei einer Temperatur zwischen 110 °C und 200 °C aus einer Oxidationszone entnommen wird.

7. Verfahren nach Anspruch 5, wobei die Gegenstrom-Wasser-Waschzone (120) 2 bis 4 Stufen von Wasser-Gegenstrom-Waschungen aufweist.

8. Verfahren nach Anspruch 5, wobei die Gegenstrom-Lösungsmittel-Waschzone (80) 2 bis 4 Stufen von Lösungsmittel-Gegenstrom-Waschungen aufweist.

9. Verfahren nach Anspruch 7, wobei die Gegenstrom-Lösungsmittel-Waschzone (80) 2 bis 4 Stufen von Lösungsmittel-Gegenstrom-Waschungen aufweist.

## Revendications

1. Procédé de production d'un gâteau d'acide carboxylique séché (170), ledit procédé comprenant :
(a) l'élimination, dans une zone de séparation solide-liquide (40), d'impuretés d'une suspension épaisse d'acide carboxylique (30) pour former un produit sous forme de suspension épaisse ou de gâteau (70) et un courant de liqueur mère (60), dans lequel ladite suspension épaisse d'acide carboxylique (30) est produite par oxydation, dans une zone d'oxydation, d'une charge de départ aromatique ;
et dans lequel ladite zone de séparation solide-liquide (40) comprend au moins un séparateur solide-liquide qui fonctionne aux températures de 50°C à 200°C ;
(b) l'ajout de solvant dans une zone de lavage au solvant à contre-courant (80) audit produit sous forme de suspension épaisse ou de gâteau (70) pour produire un gâteau d'acide carboxylique avec le solvant (110) et un courant de liqueur mère de solvant (100) ; dans lequel le solvant comprend un acide monocarboxylique aliphatique contenant de 2 à 6 atomes de carbone, ou de l'acide benzoïque, et des mélanges de ces composés avec de l'eau ;
et dans lequel la zone de lavage au solvant à contre-courant (80) comprend au moins un dispositif de séparation solide-liquide qui fonctionne dans un intervalle de température de 40°C à 155°C ;
(c) éventuellement, l'ajout d'eau dans une zone de lavage à l'eau à contre-courant (120) audit gâteau d'acide carboxylique avec le solvant (110) pour produire un gâteau d'acide carboxylique humidifié par l'eau (150) et un courant de liqueur de sous-produit de solvant/eau (140) ;
dans lequel ladite zone de lavage à contre-courant comprend un dispositif de séparation solide-liquide qui fonctionne dans un intervalle de température de 40°C à 155°C ;
(d) le séchage dudit gâteau d'acide carboxylique humidifié par l'eau (150) ou dudit gâteau d'acide carboxylique avec le solvant (110) dans une zone de séchage (160) pour former ledit gâteau d'acide carboxylique séché (170).

2. Procédé selon la revendication 1, dans lequel ledit acide carboxylique est choisi dans le groupe constitué d'acide téréphtalique, d'acide isophtalique, d'acide naphtalène dicarboxylique, d'acide trimellitique et de mélanges de ceux-ci.

3. Procédé selon la revendication 1, dans lequel ledit acide carboxylique est l'acide téréphtalique.

4. Procédé selon la revendication 3, dans lequel ladite suspension épaisse d'acide carboxylique brute (30) comprenant de l'acide téréphtalique, un catalyseur, de l'acide acétique et des impuretés, est extraite à une température située entre 110 °C et 200 °C à partir d'une zone d'oxydation.

5. Procédé selon la revendication 1 pour la production d'un gâteau d'acide téréphtalique séché (170), ledit procédé comprenant :
(a) l'élimination dans une zone de séparation solide-liquide (40) d'impuretés d'une suspension épaisse d'acide téréphtalique (30) pour former un produit d'acide téréphtalique sous forme de suspension épaisse ou de gâteau (70) et un courant de liqueur mère (60),
(b) l'ajout de solvant dans une zone de lavage au solvant à contre-courant (80) audit produit d'acide téréphtalique sous forme de suspension épaisse ou de gâteau (70) pour produire un gâteau d'acide téréphtalique avec le solvant (110) et un courant de liqueur mère de solvant (100) ;
(c) éventuellement, l'ajout d'eau dans une zone de lavage à l'eau à contre-courant (120) audit gâteau d'acide téréphtalique avec le solvant (110) pour produire un gâteau d'acide téréphtalique humidifié par l'eau (150) et un courant de liqueur de sous-produit de solvant/eau (140) ;
(d) le séchage dudit gâteau d'acide téréphtalique humidifié par l'eau (150) ou dudit gâteau d'acide téréphtalique avec le solvant (110) dans une zone de séchage (160) pour former ledit gâteau d'acide carboxylique séché (170).

6. Procédé selon la revendication 5, dans lequel ladite suspension épaisse d'acide carboxylique brut (30) comprenant de l'acide téréphtalique, un catalyseur, de l'acide acétique, et des impuretés est soutirée à une température située entre 110°C et 200°C à partir d'une zone d'oxydation.

7. Procédé selon la revendication 5, dans lequel ladite zone de lavage au solvant à contre-courant (80) comprend de 2 à 4 étapes de lavages à l'eau à contre-courant.

8. Procédé selon la revendication 5, dans lequel ladite zone de lavage au solvant à contre-courant (80) comprend de 2 à 4 étapes de lavages au solvant à contre-courant.

9. Procédé selon la revendication 7, dans lequel ladite zone de lavage au solvant à contre-courant (80) comprend de 2 à 4 étapes de lavages au solvant à contre-courant.
